# EUROPÄISCHE PATENTSCHRIFT

(11) **EP 2 338 422 B1**
(45) Veröffentlichungstag und Bekanntmachung des Hinweises auf die Patenterteilung: **15.02.2017**
(21) Anmeldenummer: 10196214.0
(22) Anmeldetag: 21.12.2010
(51) Int. Cl.: A61B 17/16

(54) **Modulare Prothesenraspel**
Modular prosthetic rasp
Râpe de prothèse modulaire

(30) Priorität: 23.12.2009 DE 102009059314; 15.02.2010 DE 102010000419
(43) Veröffentlichungstag der Anmeldung: 29.06.2011
(73) Patentinhaber: AESCULAP AG, 78532 Tuttlingen (DE)
(72) Erfinder: Fritzsche, Jörg, 78665, Frittlingen (DE); Wallstein, Stefan, 78576, Emmingen-Liptingen (DE)
(74) Vertreter: Hoeger, Stellrecht & Partner Patentanwälte mbB

(56) Entgegenhaltungen:
- WO-A1-90/03764
- DE-A1-102008 020 192
- DE-A1-102008 020 199
- US-A1- 2007 225 821

## Beschreibung

Die vorliegende Erfindung betrifft eine modulare Prothesenraspel mit einem Raspelkörper, welcher einen Raspelgrundkörper und mindestens einen mit dem Raspelgrundkörper verbindbaren modularen Raspelkörperteil umfasst, wobei der Raspelgrundkörper eine Raspelkörperteilaufnahme aufweist zum Aufnehmen des mindestens einen Raspelkörperteils in einer Verbindungsstellung, in welcher der Raspelgrundkörper und der mindestens eine Raspelkörperteil miteinander verbunden sind und in welcher der Raspelkörper eine teilweise vom Raspelgrundkörper und teilweise vom mindestens einen Raspelkörperteil definierte Außenkontur definiert, wobei eine Verbindungseinrichtung vorgesehen ist zum lösbaren Verbinden des Raspelgrundkörpers und des mindestens einen Raspelkörperteils in der Verbindungsstellung, wobei sich die Raspelkörperteilaufnahme ausgehend von einem ein distales Ende der Raspelkörperteilaufnahme definierenden Einschnitt an einer lateralen Seite des Raspelgrundkörpers bis zu einem proximalen Ende desselben erstreckt.

Eine derartige modulare Prothesenraspel ist beispielsweise aus der DE 10 2008 020 192 A1 bekannt. Sie dient insbesondere dem Zweck, in einem Femur eines Patienten vor dem Einsetzen eines Prothesenschafts einer Hüftgelenkendoprothese eine Knochenkavität und mit dem modularen Raspelkörperteil eine schlitzförmige Ausnehmung zur Aufnahme eines finnenartigen Vorsprungs am Prothesenschaft zu präparieren. Eine weitere modulare Prothesenraspel der eingangs beschriebenen Art ist ferner aus der DE 10 2008 020 199 A1 bekannt.

Nachteilig bei diesen bekannten modularen Prothesenraspeln ist jedoch, dass beim Einführen des Raspelgrundkörpers in den Markraum des Femurs nach einer Teilresektion des Trochanters letzterer in nicht gewünschter Weise teilweise geschädigt wird, da der Raspelgrundkörper lateralseitig aufgrund seiner äußeren Kontur mit dem Trochanter in Kontakt kommen und diesen sowie umliegendes Weichteilgewebe verletzen kann.

Es ist daher Aufgabe der vorliegenden Erfindung, eine modulare Prothesenraspel der eingangs beschriebenen Art so zu verbessern, dass eine Traumatisierung des Patienten beim Einführen des Raspelgrundkörpers in eine Knochenkavität minimiert wird.

Diese Aufgabe wird bei einer modularen Prothesenraspel der eingangs beschriebenen Art erfindungsgemäß dadurch gelöst, dass die Raspelkörperteilaufnahme seitlich, nämlich in anteriorer und posteriorer Richtung weisend, und proximalseitig geöffnet ist, sodass der Raspelkörperteil die Raspelkörperteilaufnahme in der Verbindungsstellung vollständig ausfüllt, und dass eine erste Tangentialebene der lateralen Seite der Außenkontur im Bereich des Einschnitts und eine zweite Tangentialebene an eine von der Raspelkörperteilaufnahme definierte und vom Raspelgrundkörper weg weisende Raspelkörperteilanlagefläche im Bereich des distalen Endes der Raspelkörperteilaufnahme einen Einschnittwinkel einschließen, welcher kleiner als 85° ist.

Durch einen Einschnittwinkel, welcher kleiner als 85° ist, ist es möglich, die Raspelkörperteilaufnahme derart zu formen, dass der Raspelgrundkörper beim Einführen insbesondere in den Markraum eines Femurs zum Präparieren einer Kavität für einen Hüftgelenkendoprothesenschaft nicht mehr mit dem Trochanter in Kontakt kommen kann. Dadurch wird eine unerwünschte Verletzung nicht nur des Trochanters, sondern auch von umliegendem Weichteilgewebe verhindert. Die Raspelkörperteilaufnahme bildet somit eine laterale Aussparung, welche einerseits eine Traumatisierung von Weichteilen und Knochen deutlich vermindert und andererseits mit geringem Kraftaufwand eine optimale Positionierung des eigentlichen Implantats ermöglicht. Gemäß der Erfindung ist eine Verbindungseinrichtung vorgesehen zum lösbaren Verbinden des Raspelgrundkörpers und des mindestens einen Raspelkörperteils in der Verbindungsstellung. Die Verbindungseinrichtung ermöglicht es, den Raspelgrundkörper und das mindestens eine Raspelkörperteil gezielt auf einfache Weise sicher miteinander zu verbinden.

Günstigerweise weist der Einschnittwinkel einen Wert in einem Bereich von 20° bis 70° auf. Günstig ist es, wenn der Wert in einem Bereich von 35° bis 55° liegt. Vorzugsweise beträgt der Einschnittwinkel etwa 45°. Ein Einschnittwinkel mit Werten in den angegebenen Bereichen ermöglicht die Ausbildung eines stabilen Raspelgrundkörpers und zudem einer Raspelkörperteilaufnahme, welche das Einführen des Raspelgrundkörpers in die Knochenkavität ermöglicht, ohne Weichteilgewebe sowie den Trochanter zu schädigen.

Günstig ist es ferner, wenn die Raspelkörperteilanlagefläche eine stetige oder im Wesentlichen stetige Fläche definiert. Darunter ist insbesondere zu verstehen, dass diese Fläche keine Knicke aufweist und, im Sinne einer mathematischen Definition, nicht nur stetig, sondern auch differenzierbar ist.

Um eine besonders stabile Prothesenraspel ausbilden zu können, insbesondere dann, wenn der Raspelgrundkörper und der modulare Raspelkörperteil die Verbindungsstellung einnehmen, ist es vorteilhaft, wenn der mindestens eine Raspelkörperteil eine Raspelgrundkörperanlagefläche aufweist, welche in der Verbindungsstellung flächig oder im Wesentlichen flächig an der Raspelkörperteilanlagefläche anliegt. Durch die flächige oder im Wesentlichen flächige Anlage des Raspelkörperteils am Raspelgrundkörper kann eine besonders gute Abstützung erreicht werden.

Die Herstellung der Prothesenraspel kann auf einfache Weise dadurch vereinfacht werden, dass die Raspelkörperteilanlagefläche einen ebenen oder im Wesentlichen ebenen ersten Raspelkörperteilanlageflächenbereich umfasst.

Noch einfacher herstellen lässt sich die Prothesenraspel, wenn sich der erste Raspelkörperteilanlageflächenbereich hin zu einem proximalen Ende der Raspelkörperteilaufnahme erstreckt. Insbesondere kann er sich ganz bis zum proximalen Ende der Raspelkörperteilaufnahme erstrecken.

Günstig ist es, wenn der erste Raspelkörperteilanlageflächenbereich und eine Längsachse des Raspelgrundkörpers einen Neigungswinkel definieren, welcher einen Wert in einem Bereich von 0° bis 25° aufweist. Vorzugsweise liegt der Neigungswinkel in einem Bereich von 0° bis 10°. Besonders vorteilhaft ist es, wenn der Neigungswinkel etwa 5° beträgt. Durch das Vorsehen eines Neigungswinkels in den angegebenen Bereichen kann das Risiko eines Kontakts zwischen dem Raspelgrundkörper und dem Trochanter sowie umliegendem Weichteilgewebe beim Einführen des Raspelgrundkörpers in den Markraum des Femurs noch weiter verringert werden. Insbesondere kann der Neigungswinkel zwischen der Längsachse und dem ersten Raspelkörperteilanlageflächenbereich definiert werden, wenn der Raspelkörperteilanlageflächenbereich ausgehend von seinem distalen Ende in medialer Richtung geneigt ist, also insbesondere wenn ein Abstand des ersten Raspelkörperteilanlageflächenbereichs von einer lateralen Seitenfläche des Raspelkörpers in proximaler Richtung, also auf das proximale Ende des Raspelkörpers hin, zunimmt.

Das Einführen des Raspelgrundkörpers in die Kavität des Femurs kann noch weiter verbessert werden, wenn die Raspelkörperteilanlagefläche einen zweiten Raspelkörperteilanlageflächenbereich umfasst, welcher kontinuierlich gekrümmt ist. Vorzugsweise geht der zweite Raspelkörperteilanlageflächenbereich im mathematischen Sinne stetig in den sich an ihn proximalseitig anschließenden ersten Raspelkörperteilanlageflächenbereich über.

Besonders einfach herstellen lässt sich der Raspelgrundkörper, wenn der zweite Raspelkörperteilanlageflächenbereich einen konstanten oder im Wesentlichen konstanten Krümmungsradius aufweist. Beispielsweise kann er so mit einem einfachen rotierenden Metallbearbeitungswerkzeug, beispielsweise einem Fräser, präpariert werden.

Günstig ist es, wenn der Krümmungsradius einen Wert in einem Bereich von 5 mm bis 25 mm aufweist. Die Stabilität des Raspelgrundkörpers lässt sich beispielsweise weiter dadurch verbessern, dass der Krümmungsradius einen Wert in einem Bereich von 10 mm bis 20 mm aufweist.

Auf besonders einfache Weise lässt sich der Raspelgrundkörper in eine Kavität eines Femurs einführen, wenn der zweite Raspelkörperteilanlageflächenbereich sich bis zum distalen Ende der Raspelkörperteilaufnahme erstreckt. Insbesondere ist es für die Herstellung der Prothesenraspel vorteilhaft, wenn ein distaler, sich bis zum distalen Ende der Raspelkörperteilaufnahme erstreckender Teilbereich des zweiten Raspelkörperteilanlageflächenbereichs eben oder im Wesentlichen eben ausgebildet ist. Zwischen diesem ebenen Teilbereich und dem ersten Raspelkörperteilanlageflächenbereich kann dann beispielsweise ein gekrümmter Teilbereich des zweiten Raspelkörperteilanlageflächenbereichs ausgebildet sein.

Besonders einfach herstellen lässt sich der Raspelgrundkörper, wenn zwei voneinander weg weisende Seitenflächen eines ersten Seitenflächenpaars desselben parallel oder im Wesentlichen parallel zueinander verlaufen. Alternativ kann es auch günstig sein, wenn sie etwas gegeneinander geneigt sind, so dass sich in distaler Richtung ein Abstand zwischen ihnen verringert.

Günstig ist es, wenn die zwei Seitenflächen des ersten Seitenflächenpaars eben oder im Wesentlichen eben sind. Die jeweiligen Flächen können dann besonders einfach hergestellt werden.

Gemäß einer weiteren bevorzugten Ausführungsform der Erfindung kann vorgesehen sein, dass zwei voneinander weg weisende Seitenflächen eines zweiten Seitenflächenpaars des Raspelgrundkörpers mindestens teilweise eben ausgebildet sind und ihre ebenen Bereiche gegeneinander um einen Schaftwinkel geneigt sind, welcher Schaftwinkel einen Wert in einem Bereich von 0° bis 15° aufweist. Vorzugsweise weist der Schaftwinkel einen Wert in einem Bereich von 0° bis 8° auf. Vorzugsweise sind laterale und mediale Seitenflächen relativ zueinander um den Schaftwinkel geneigt. Auf diese Weise kann eine optimierte Fixierung des Prothesenschafts in der zu präparierenden Knochenkavität erreicht werden, und zwar aufgrund einer Konusklemmung beziehungsweise eines Preßsitzes.

Die Herstellung des Raspelgrundkörpers lässt sich weiter vereinfachen, wenn beispielsweise die zwei Seitenflächen des zweiten Seitenflächenpaars ausgehend vom Einschnitt bis zu einem distalen Endbereich des Raspelgrundkörpers eben oder im Wesentlichen eben sind. Beispielsweise kann so auch eine mediale Seitenfläche ausgehend vom Einschnitt in proximaler Richtung gekrümmt sein und sich so optimal an eine natürliche Kavität des Knochens anschmiegen.

Besonders einfach herstellen lässt sich die Prothesenraspel, wenn die zwei Seitenflächen des zweiten Seitenflächenpaars vollständig eben oder im Wesentlichen eben sind.

Zur Ausbildung einer Kavität, die einem Prothesenschaft durch Klemmung oder Preßsitz einen hervorragenden Halt bietet, ist es günstig, wenn eine Querschnittsfläche des Raspelgrundkörpers senkrecht zu einer Längsachse desselben ausgehend vom distalen Ende der Raspelkörperteilaufnahme in Richtung zu einem distalen Ende des Raspelgrundkörpers hin abnimmt.

Es ist insbesondere vorteilhaft, wenn bei einer Prothesenraspel der eingangs beschriebenen Art eine Querschnittsfläche des Raspelgrundkörpers senkrecht zu einer Längsachse desselben ausgehend vom distalen Ende der Raspelkörperteilaufnahme in Richtung zu einem distalen Ende des Raspelgrundkörpers hin abnimmt. So kann auf einfache und sichere Weise ein perfekter Preßsitz des eigentlichen Prothesenschafts in der vorbereiteten Knochenkavität sichergestellt werden.

Vorteilhaft ist es, wenn eine Querschnittsfläche des Raspelgrundkörpers senkrecht zu einer Längsachse desselben ausgehend vom distalen Ende der Raspelkörperteilaufnahme in Richtung zu einem proximalen Ende des Raspelgrundkörpers hin bis zu einem Minimum abnimmt und vom Minimum bis zum proximalen Ende hin wieder zunimmt. Diese besondere Formgebung eines proximalen Endbereichs des Raspelgrundkörpers ermöglicht es, diesen so auszubilden, dass beim Einführen desselben in den Markraum des Femurs Weichteilgewebe sowie der Trochanter nicht beschädigt werden.

Günstig kann es ferner sein, wenn bei einer Prothesenraspel der eingangs beschriebenen Art eine Querschnittsfläche des Raspelgrundkörpers senkrecht zu einer Längsachse desselben ausgehend vom distalen Ende der Raspelkörperteilaufnahme in Richtung zu einem proximalen Ende des Raspelgrundkörpers hin bis zu einem Minimum abnimmt und vom Minimum bis zum proximalen Ende hin wieder zunimmt. In analoger Weise wie eben beschrieben ermöglicht diese Ausgestaltung des Raspelgrundkörpers das Einführen desselben in eine Knochenkavität eines Femur ohne oder im Wesentlichen ohne eine Traumatisierung umliegenden Weichteilgewebes sowie des Trochanters.

Gemäß einer weiteren bevorzugten Ausführungsform der Erfindung kann vorgesehen sein, dass eine Funktion der Querschnittsfläche des Raspelgrundkörpers senkrecht zur Längsachse ausgehend vom distalen Ende der Raspelkörperteilaufnahme in Richtung zu einem proximalen Ende des Raspelgrundkörpers hin in Abhängigkeit des Abstandes der die Querschnittsfläche definierenden Schnittebene vom distalen Ende der Raspelkörperteilaufnahme eine mathematisch stetige Funktion bildet. Vorzugweise handelt es sich dabei um eine stetige und differenzierbare Funktion, die also keine Knicke oder Sprünge aufweist. Eine derartige Funktion weist vorzugsweise eine Rechtskrümmung auf, was einem positiven Wert der zweiten Ableitung der Funktion entspricht.

Besonders günstig ist es, wenn das Minimum vom distalen Ende der Raspelkörperteilaufnahme einen Abstand aufweist, welcher mindestens dem 0,1fachen einer Länge der Raspelkörperteilaufnahme parallel zur Längsachse entspricht. Durch einen solchen Abstand ist sichergestellt, dass ein sanfter Einschnitt der Raspelkörperteilaufnahme gegeben ist, der es ermöglicht, eine Verletzung von umliegendem Weichteilgewebe sowie des Trochanters beim Einführen des Raspelgrundkörpers in den Markraum des Knochen zu vermeiden.

Vorteilhaft kann es ferner auch sein, wenn der Abstand mindestens dem 0,2fachen der Länge der Raspelkörperteilaufnahme entspricht. Günstig ist es zudem, wenn der Abstand mindestens dem 0,3fachen der Länge der Raspelkörperteilaufnahme entspricht. Bei bevorzugten Ausführungsformen kann das Minimum einen Abstand vom distalen Ende der Raspelkörperteilaufnahme aufweisen, welcher etwa dem 0,4 bis 0,5fachen der Länge der Raspelkörperteilaufnahme entspricht. Das Minimum etwa im Mittelbereich der Raspelkörperteilaufnahme vorzusehen ermöglicht insbesondere eine noch weiter verbesserte Formgebung, die unabhängig von einer Position des Raspelgrundkörpers beim Eindrehen, das heißt beim Einführen in die Kavität des Markraums des Femurs, einen zur Vermeidung einer Traumatisierung des Trochanters und umliegenden Weichteilgewebes erforderlichen Abstand aufweist.

Zur Vermeidung von Verletzungen beim Einführen des Raspelschafts in die Knochenkavität ist es günstig, wenn der Raspelgrundkörper ein distales Ende aufweist, welches eine stumpfe Spitze bildet.

Um den Markraum des Knochens in eine Form zu bringen, welche zur Aufnahme des Prothesenschafts optimal passt, ist es vorteilhaft, wenn eine oder mehrere Seitenflächen des Raspelgrundkörpers und/oder des mindestens einen Raspelkörperteils mindestens teilweise mit Raspelzähnen zur Knochenbearbeitung versehen sind. Vorzugsweise sind die Raspelkörperteilanlagefläche sowie die Raspelgrundkörperanlagefläche raspelzahnfrei. Für eine optimierte Bearbeitung ist es zudem günstig, wenn alle Seitenflächen, die die äußere Kontur des Raspelkörpers definieren, mit Raspelzähnen versehen sind. Das distale Ende des Raspelgrundkörpers ist vorzugsweise raspelzahnfrei, um beim Einführen des Raspelgrundkörpers in den Markraum diesen und insbesondere die Kortikalis nicht in unerwünschter Weise zu verletzen oder aufzuweiten.

Zur Verbesserung einer Handhabung der Prothesenraspel ist es vorteilhaft, wenn am Raspelgrundkörper im Bereich seines proximalen Endes ein Kupplungselement zum lösbaren Verbinden mit einem Raspelgriff angeordnet oder ausgebildet ist. Das Kupplungselement kann männlich oder weiblich ausgebildet sein. Mittels eines Raspelgriffs kann die Prothesen raspel in gewünschter Weise manipuliert werden zum Präparieren der Knochenkavität.

Des Weiteren ist es günstig, wenn an dem mindestens einen Raspelkörperteil im Bereich seines proximalen Endes ein Kupplungselement zum lösbaren Verbinden mit einem Handgriff angeordnet oder ausgebildet ist. Beispielsweise kann so nach Einführen des Raspelgrundkörpers in die Knochenkavität in einem zweiten Schritt der Raspelkörperteil unabhängig vom Raspelgrundkörper eingeführt und beispielsweise mit dem Raspelgrundkörper verbunden werden, um die Kavität im Knochen, insbesondere im Bereich des Trochanters in gewünschter Weise zu präparieren.

Auf einfache Weise lässt sich die Verbindungseinrichtung ausbilden, wenn sie erste und zweite Verbindungselemente umfasst, welche einerseits am Raspelgrundkörper und andererseits am mindestens einen Raspelkörperteil angeordnet oder ausgebildet sind und in der Verbindungsstellung miteinander in Eingriff stehen. Insbesondere können die Verbindungselemente ausgebildet sein zur Ausbildung einer kraft- und/oder formschlüssigen Verbindung des Raspelgrundkörpers und des mindestens einen Raspelkörperteils miteinander.

Vorteilhaft ist es, wenn der Raspelgrundkörper ein weibliches oder im Wesentlichen weibliches Verbindungselement umfasst und wenn der mindestens eine Raspelkörperteil ein korrespondierendes männliches oder im Wesentlichen männliches Verbindungselement umfasst. Diese Ausgestaltung hat insbesondere den Vorteil, dass keine oder im Wesentlichen keine Teile von der Raspelkörperteilanlagefläche des Raspelgrundkörpers abstehen beziehungsweise abstehen müssen, die beim Einführen des Raspelgrundkörpers in den Markraum des Knochens in unerwünschter Weise zu einer Traumatisierung von umliegendem Weichteilgewebe oder des Trochanters führen können. Günstigerweise ist das weibliche Verbindungselement in Form einer Ausnehmung ausgebildet und das männliche Verbindungselement in Form eines korrespondierenden Vorsprungs. Insbesondere ist es vorteilhaft, wenn die beiden Verbindungselemente formschlüssig oder zumindest teilweise formschlüssig in der Verbindungsstellung miteinander in Eingriff stehen, wodurch eine definierte und sichere Verbindung zwischen dem Raspelgrundkörper und dem mindestens einem Raspelkörperteil hergestellt werden kann.

Gemäß einer weiteren bevorzugten Ausführungsform der Erfindung kann es bei einer Prothesenraspel der eingangs beschriebenen Art vorteilhaft sein, wenn eine Verbindungseinrichtung zum lösbaren Verbinden des Raspelgrundkörpers und des mindestens einen Raspelkörperteils in der Verbindungsstellung vorgesehen ist, wenn die Verbindungseinrichtung erste und zweite Verbindungselemente umfasst, welche einerseits am Raspelgrundkörper und andererseits am mindestens einen Raspelkörperteil angeordnet oder ausgebildet sind und in der Verbindungsstellung miteinander in Eingriff stehen, wenn der Raspelgrundkörper ein weibliches oder im Wesentlichen weibliches Verbindungselement umfasst und wenn der mindestens eine Raspelkörperteil ein korrespondierendes männliches oder im Wesentlichen männliches Verbindungselement umfasst. Eine Prothesenraspel in dieser Weise auszubilden hat insbesondere den Vorteil, dass zum Verbinden des Raspelgrundkörpers und des mindestens einen Raspelkörperteils keine Teile, insbesondere keine Verbindungselemente, von der Raspelkörperteilaufnahme in Richtung auf das mindestens eine Raspelkörperteil vorstehen müssen. Insbesondere, wenn die Raspelkörperteilaufnahme eine vom Raspelgrundkörper weg weisende Raspelkörperteilanlagefläche definiert, kann diese vorsprungfrei ausgebildet werden. Genau in diesem Bereich ist dies auch besonders günstig, da so auf einfache Weise ein in Kontakt Treten mit umliegendem Weichteilgewebe sowie mit dem Trochanter beim Einführen des Raspelgrundkörpers in den Markraum des zu bearbeitenden Knochens vermieden werden kann.

Besonders einfach herstellen lässt sich die Verbindungseinrichtung, wenn das weibliche Verbindungselement eine Bohrung oder ein Sackloch umfasst. Das männliche Verbindungselement kann dann beispielsweise in Form eines korrespondierenden Vorsprungs oder Zapfens ausgebildet sein, welcher formschlüssig in das weibliche Verbindungselement einführbar, insbesondere einschiebbar, ist. Vorzugsweise ist die Verbindungseinrichtung derart ausgebildet, dass der Grundkörper und das mindestens eine Raspelkörperteil durch eine Verschiebung relativ zueinander parallel zu einer von mindestens einem der Verbindungselemente definierten Achse miteinander in Eingriff gebracht werden können.

Damit das mindestens eine Raspelkörperteil am Raspelgrundkörper in definierter und sicherer Weise gehalten werden kann, ist es günstig, wenn das weibliche Verbindungselement einen lateralen Führungsschlitz aufweist, welcher sich bis zur Raspelkörperteilanlagefläche erstreckt, beispielsweise kann so das männliche Verbindungselement des mindestens einen Raspelkörperteils den Führungsschlitz durchgreifen und mit einem vorspringenden Teilbereich in insbesondere eine durch das weibliche Verbindungselement ausgebildete Hinterschneidung eingreifen, um so den mindestens einen Raspelkörperteil in der Verbindungsstellung am Raspelgrundkörper zu halten.

Um sicherzustellen, dass der mindestens eine Raspelkörperteil und der Raspelgrundkörper sich nicht in unerwünschter Weise voneinander lösen können, ist es vorteilhaft, wenn eine Sicherungseinrichtung zum Sichern einer Verbindung des Raspelgrundkörpers und des mindestens einen Raspelkörperteils in der Verbindungsstellung vorgesehen ist.

Auf einfache Weise herstellen lässt sich die Sicherungseinrichtung, wenn sie erste und zweite Sicherungselemente umfasst, welche einerseits am Raspelgrundkörper und andererseits am mindestens einen Raspelkörperteil angeordnet oder ausgebildet sind und in einer Sicherungsstellung miteinander in Eingriff stehen. In der Sicherungsstellung stehen somit die ersten und zweiten Sicherungselemente miteinander in Eingriff sowie die männlichen und weiblichen Verbindungselemente, welche die Verbindungsstellung einnehmen.

Um eine besonders kompakte Prothesenraspel ausbilden zu können, ist es günstig, wenn das erste Sicherungselement am oder im Bereich des weiblichen Verbindungselements angeordnet oder ausgebildet ist und wenn das zweite Sicherungselement am oder im Bereich des männlichen Verbindungselements angeordnet oder ausgebildet ist. Beispielsweise kann ein Sicherungselement in Form einer Ausnehmung und das korrespondierende andere Sicherungselements in Form eines Vorsprungs ausgebildet sein, wobei die Sicherungselemente vorzugsweise relativ zueinander beweglich angeordnet oder ausgebildet sind, um auf eine einfache Weise miteinander in Eingriff und wieder außer Eingriff gebracht werden zu können.

Für die Handhabung der Prothesenraspel ist es insbesondere vorteilhaft, wenn die Sicherungseinrichtung in Form einer Rastverbindungseinrichtung ausgebildet ist und wenn die ersten und zweiten Sicherungselemente in Form von Rastelementen ausgebildet sind, welche in der Sicherungsstellung miteinander rastend in Eingriff stehen. Eine Rastverbindungseinrichtung ermöglicht es insbesondere, den Raspelgrundkörper und den mindestens einen Raspelkörperteil in der Verbindungsstellung automatisch zu sichern, da beim Überführen des Raspelgrundkörpers und des mindestens einen Raspelkörperteils in die Verbindungsstellung die Verbindungselemente miteinander rastend in Eingriff treten können. Vorzugsweise ist dabei ein Rastvorsprung vorgesehen, welcher in eine Rastnehmung in der Sicherungsstellung eingreifen kann. Der Rastvorsprung ist vorteilhafterweise beweglich gelagert am mindestens einen Raspelkörperteil oder am Raspelgrundkörper. Ferner kann vorgesehen sein, die ersten und zweiten Sicherungselemente federnd gegeneinander vorzuspannen, so dass sie beispielsweise bei der Auslenkung aus der Sicherungsstellung wieder automatisch in diese zurückgehen können.

Vorteilhaft ist es, wenn der Raspelgrundkörper und der mindestens eine Raspelkörperteil in der Verbindungsstellung relativ zueinander bewegbar sind. Diese Ausgestaltung ermöglicht es, die Verbindungsseinrichtung insbesondere auch als Führungseinrichtung zu nutzen, um eine Relativbewegung des mindestens einen Raspelkörperteils und des Raspelgrundkörpers in gezielter Weise zu führen.

Um die Raspelkörperteilaufnahme in einer Weise auszubilden, die groß genug ist, um einen Kontakt zwischen der Raspelkörperteilanlagefläche und dem Trochanter zu vermeiden, ist es günstig, wenn die zweite Tangentialebene und der erste Raspelkörperteilanlageflächenbereich einen stumpfen Öffnungswinkel einschließen.

Vorzugsweise weist der stumpfe Öffnungswinkel einen Wert von 95° bis 160° auf. Günstig kann es ferner sein, wenn der stumpfe Öffnungswinkel einen Wert in einem Bereich von 105° bis 150° aufweist.

Beim Einführen des Raspelgrundkörpers in den Markraum eines Knochens, insbesondere eines Femurs, kann der Raspelgrundkörper relativ zum Knochen sukzessive verschwenkt werden. Insbesondere kann sein lateraler proximaler Endbereich in Richtung auf den Trochanter hin verschwenkt werden, wenn ein distaler Endbereich des Raspelgrundkörpers in Form eines langgestreckten, geradlinig ausgebildeten Schafts ausgebildet ist. Um sicherzustellen, dass umliegendes Weichteilgewebe sowie der Trochanterbereich beim Einführen des Raspelgrundkörpers nicht verletzt werden, ist es günstig, wenn eine Länge des ersten Raspelkörperteilanlageflächenbereichs parallel oder im Wesentlichen parallel zu einer Längsachse des Raspelgrundkörpers etwa dem 0,4fachen bis 0,75fachen der Länge der Raspelkörperteilaufnahme entspricht. Vorzugsweise liegt der Wert in einem Bereich von 0,6 bis 0,7. Besonders bevorzugt ist ein Längenverhältnis im Bereich von 0,62 bis 0,66.

Gemäß einer weiteren bevorzugten Ausführungsform der Erfindung kann es vorteilhaft sein, wenn die Querschnittsfläche des Raspelgrundkörpers im Minimum einen Wert aufweist, welcher dem 0,5fachen bis 0,8fachen der Querschnittsfläche im Bereich des Einschnitts entspricht. Besonders vorteilhaft ist es, wenn die Querschnittsfläche im Bereich des Minimums dem 0,62fachen bis 0,7fachen der Querschnittsfläche im Bereich des Einschnitts entspricht. Diese Querschnittsflächenverhältnisse ermöglichen es insbesondere, der Raspelkörperteilaufnahme eine Form zu geben, die das Einführen des Raspelgrundkörpers in den zu bearbeitenden Knochen ermöglicht, ohne den Trochanter zu verletzen.

Um die Traumatisierung umliegenden Weichteilgewebes beim Einführen des Raspelgrundkörpers in den Markraum des Knochens zu minimieren, ist es vorteilhaft, wenn die Raspelkörperteilanlagefläche und an diese angrenzende Seitenflächen des Raspelgrundkörpers mindestens teilweise abgerundete Kanten aufweisen. Insbesondere kann auch die Raspelkörperteilanlagefläche vom Raspelgrundkörper weg weisend eine konvexe Außenkontur aufweisen.

Besonders stabil ausbilden lässt sich die Prothesenraspel, wenn der Raspelgrundkörper und/oder der mindestens eine Raspelkörperteil jeweils einstückig ausgebildet sind.

Die Handhabbarkeit der Prothesenraspel kann insbesondere dadurch erhöht werden, dass ein mit dem Raspelgrundkörper lösbar verbindbarer Raspelgriff vorgesehen ist.

Der Raspelgriff lässt sich mit dem Raspelgrundkörper auf einfache Weise insbesondere dadurch verbinden, wenn eine Kopplungseinrichtung zum lösbaren Koppeln des Raspelgriffs mit dem Raspelgrundkörper vorgesehen ist, welche Kopplungseinrichtung in einer Kopplungsstellung in Eingriff bringbare erste und zweite Kopplungselemente am Raspelgriff und am Raspelgrundkörper umfasst. Die Kopplungselemente können insbesondere ausgebildet sein, um in der Kopplungsstellung kraft- und/oder formschlüssig in Eingriff zu stehen. Denkbar sind insbesondere zueinander korrespondierende Vorsprünge und Ausnehmungen, die wahlweise am Raspelgrundkörper und am Raspelgriff angeordnet oder ausgebildet sein können.

Zur weiteren Verbesserung der Handhabbarkeit der Prothesenraspel ist es vorteilhaft, wenn diese einen mit dem mindestens einen Raspelkörperteil lösbar verbindbaren Handgriff umfasst. Je nach Ausgestaltung des Raspelgriffs kann dieser optional auch den Hangriff bilden, so dass er wahlweise mit dem Raspelgrundkörper und mit dem mindestens einen Raspelkörperteil verbunden werden kann. Der Handgriff weist vorzugsweise ein zum Kupplungselement des mindestens einen Raspelkörperteils korrespondierendes Kupplungselement auf.

Um insbesondere den Trochanter nach Einführen des Raspelgrundkörpers in den Markraum des Knochens in gewünschter Weise bearbeiten zu können, ist es günstig, wenn die Prothesenraspel einen mit dem Raspelgrundkörper lösbar verbindbaren Meißelteil umfasst. Vorzugsweise kann der Meißelteil mit dem Raspelgrundkörper in Eingriff gebracht und dann noch relativ zu diesem bewegt werden, so dass eine genau definierte Präparierung des Trochanters mit dem Meißelteil möglich ist.

Vorteilhaft ist es, wenn der Meißelteil mindestens ein drittes Verbindungselement umfasst, welches mit dem am Raspelgrundkörper angeordneten oder ausgebildeten Verbindungselement mindestens teilweise in Eingriff bringbar ist. Insbesondere kann das dritte Verbindungselement ausgebildet sein, um mit dem weiblichen Verbindungselement des Raspelgrundkörpers in Eingriff gebracht zu werden. Beispielsweise kann es, wenn das weibliche Verbindungselement in Form einer Bohrung oder eines Sacklochs ausgebildet ist, in Form eines korrespondierenden stabförmigen Vorsprungs ausgebildet sein.

Vorteilhafterweise bildet das vom Raspelgrundkörper umfasste Verbindungselement eine Führung für das dritte Verbindungselement zum Führen einer Bewegung des Meißelteils relativ zum Raspelgrundkörper. Wird der Raspelgrundkörper im Markraum des Knochens zunächst in seine gewünschte Form gebracht, kann dann in definierter Weise der Meißelteil relativ zum Raspelgrundkörper geführt werden, um beispielsweise den Trochanter vor einer Präparierung mit dem mindestens einen Raspelkörperteil vorzubearbeiten.

Um die Handhabung des Meißelteils zu verbessern, ist es günstig, wenn dieser mindestens ein Kupplungselement umfasst, insbesondere zum Verbinden mit einem Handgriff. Insbesondere kann das Kupplungselement ausgebildet sein, um mit dem mit dem mindestens einen Raspelkörperteil verbindbaren Handgriff verbunden zu werden. Auf diese Weise kann die Zahl der für die Handhabung der Prothesenraspel erforderlichen Handgriffe minimiert werden.

Damit eine Bearbeitung insbesondere des Trochanters mit dem Meißelteil in besonders definierter Weise erfolgen kann, ist es günstig, wenn ein Anschlag zum Begrenzen einer Bewegung des Meißelteils relativ zum Raspelgrundkörper vorgesehen ist. Insbesondere kann der Anschlag dazu dienen, eine Bewegung des Meißelteils relativ zum Raspelgrundkörper in distaler Richtung zu begrenzen.

Auf einfache Weise ausbilden lässt sich der Anschlag, wenn ein proximales Ende des Raspelgrundkörpers diesen bildet. Der Raspelgrundkörper kann dann in gewünschter Weise in den Markraum des Knochens eingeführt und der Meißelteil bis an das proximale Ende des Raspelgrundkörpers herangeführt werden, wobei bei dieser Bewegung Knochen, insbesondere der Trochanter, bearbeitet werden kann.

Die nachfolgende Beschreibung bevorzugter Ausführungsformen der Erfindung dient im Zusammenhang mit der Zeichnung der näheren Erläuterung. Es zeigen:
- Figur 1:: eine schematische Gesamtansicht eines Teils einer Prothesenraspel beim Einführen in den Markraum eines Femurknochens;

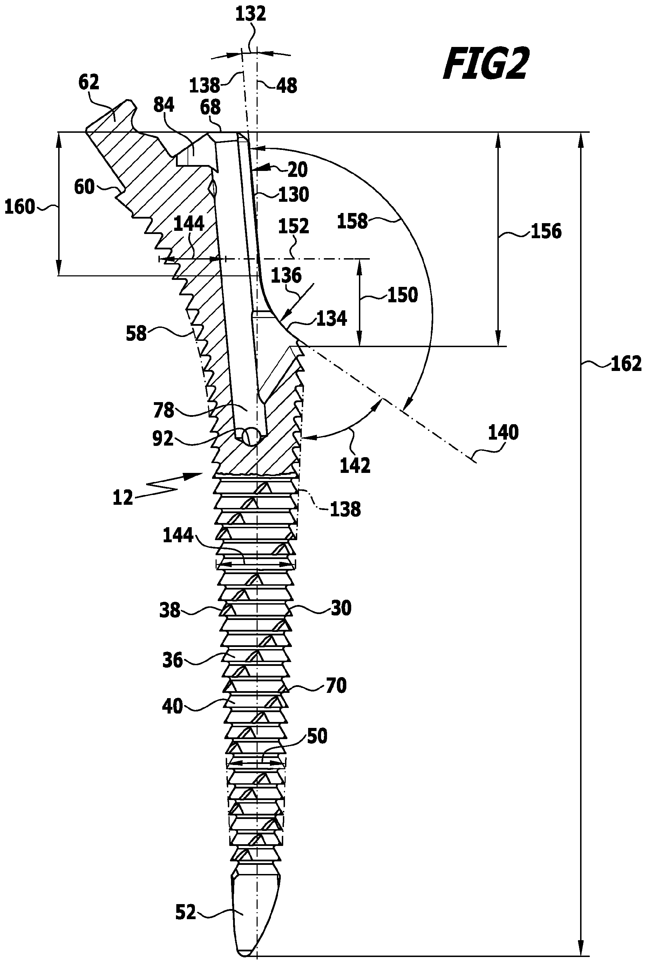

- Figur 2:: eine teilweise geschnittene Seitenansicht eines Raspelgrundköpers der Prothesenraspel;

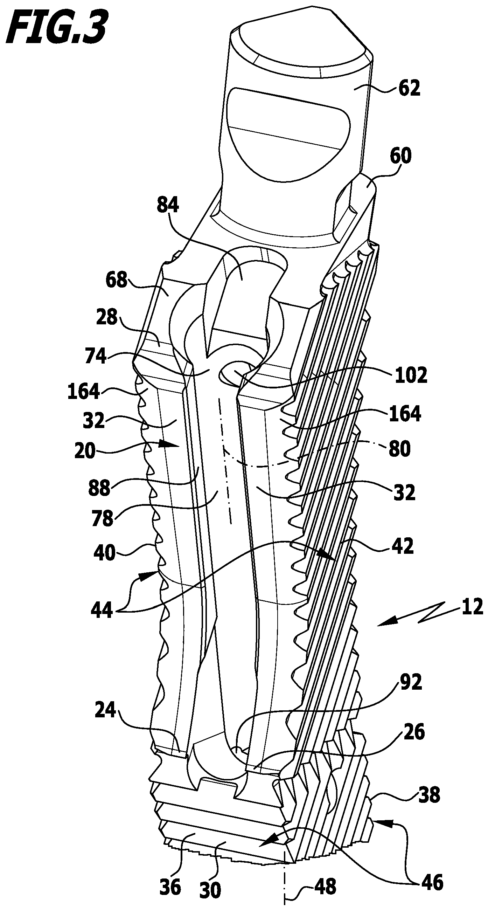

- Figur 3:: eine perspektivische Teilansicht eines proximalen Endbereichs des Raspelgrundkörpers;
- Figur 4:: eine teilweise geschnittene Seitenansicht eines proximalen Endbereichs des Raspelgrundkörpers mit angekoppeltem Raspelkörperteil;
- Figur 5:: eine vergrößerte Teilansicht der Figur 1;
- Figur 6:: eine Seitenansicht des in den Markraum des Femurs eingeführten Raspelgrundkörpers mit Bahnkurven verschiedener Raspelgrundkörper beim Einführen in den Markraum;
- Figur 7:: eine teilweise durchbrochene Seitenansicht des in den Markraum des Femurs eingeführten Raspelkörpers mit gekoppeltem Meißelteil;

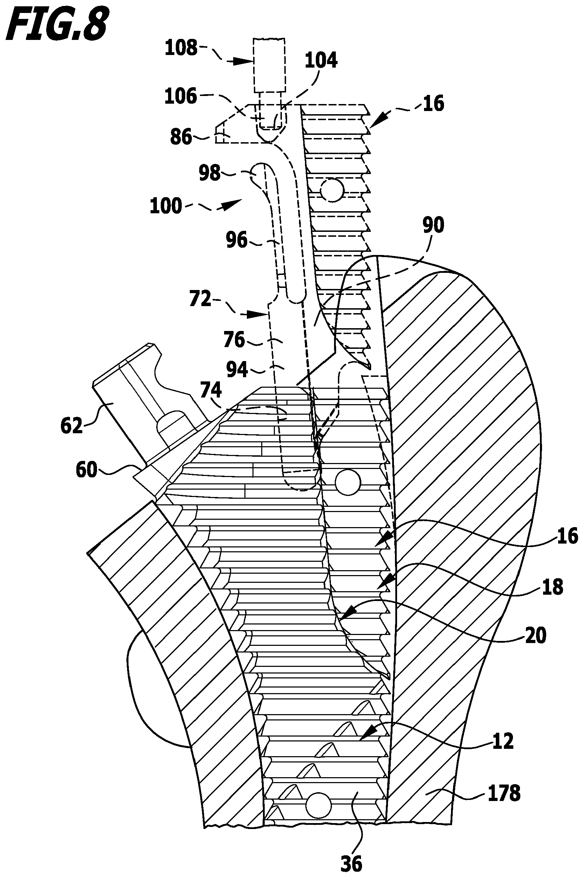

- Figur 8:: eine Ansicht ähnlich Figur 7, jedoch beim Bearbeiten des Trochanters mittels des Raspelkörperteils; und
- Figur 9:: eine graphische Darstellung normierter Querschnitte dreier Prothesenraspeln.

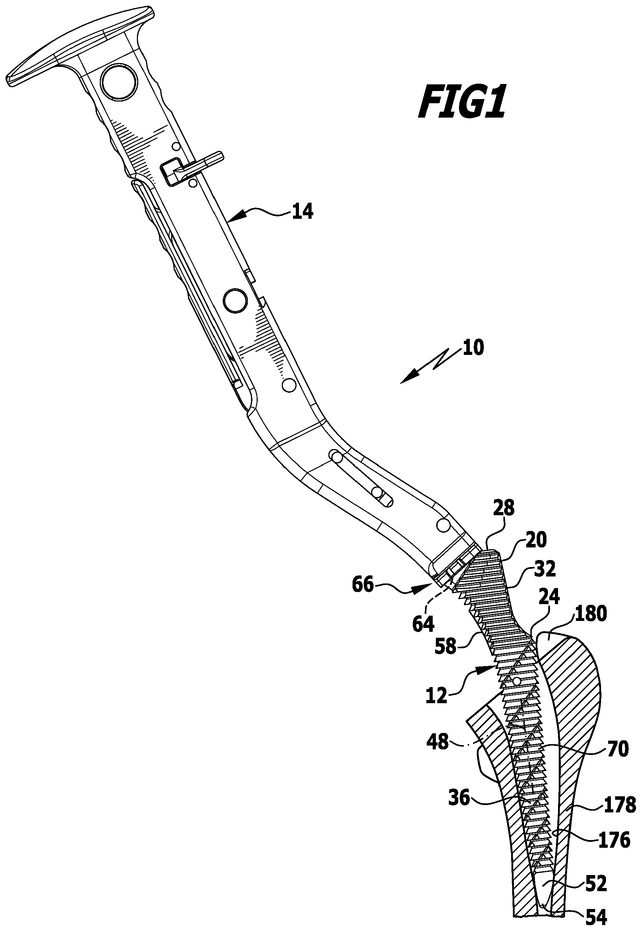

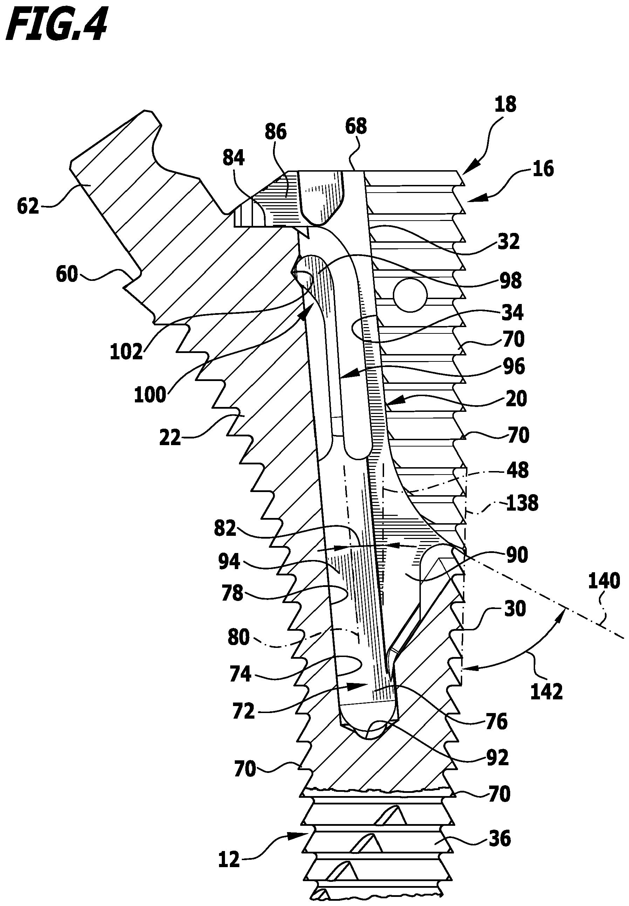

In Figur 1 ist ein erstes Ausführungsbeispiel einer insgesamt mit dem Bezugszeichen 10 bezeichneten modularen Prothesenraspel schematisch dargestellt. Sie umfasst einen Raspelgrundkörper 12 und einen mit diesem lösbar verbindbaren Raspelgriff 14. Des Weiteren umfasst die Prothesenraspel 10 einen Raspelkörperteil 16, welcher mit dem Raspelgrundkörper 12 lösbar verbindbar ist und in einer Verbindungsstellung, wie beispielhaft in Figur 4 dargestellt, mit dem Raspelgrundkörper 12 zusammen insgesamt einen Raspelkörper 18 bildet.

Der Raspelgrundkörper 12 umfasst eine Raspelkörperteilaufnahme 20 zum Aufnehmen des Raspelkörperteils 16 in der Verbindungsstellung. In dieser definieren der Raspelgrundkörper 12 sowie der Raspelkörperteil 16 jeweils teilweise eine Außenkontur 22 des Raspelkörpers 18.

Die Raspelkörperteilaufnahme 20 erstreckt sich ausgehend von einem ein distales Ende 24 derselben definierenden Einschnitt 26 an einer lateralen Seitenfläche 30 des Raspelgrundkörpers 12 bis zu einem proximalen Ende 28 desselben. Die Raspelkörperteilaufnahme 20 ist seitlich, also in anteriorer und posteriorer Richtung weisend, und proximalseitig geöffnet, so dass der Raspelkörperteil 16 die Raspelkörperteilaufnahme 20 vollständig ausfüllt. Eine vom Raspelgrundkörper 12 weg weisende Begrenzungsfläche der Raspelkörperteilaufnahme 20 definiert eine Raspelkörperteilanlagefläche 32, an welcher der Raspelkörperteil 16 mit einer korrespondierenden Raspelgrundkörperanlagefläche 34 flächig oder im Wesentlichen flächig anliegt.

Distalseitig des Einschnitts 26 ist der Raspelgrundkörper 12 in Form eines Schafts 36 ausgebildet mit vier ebenen Seitenflächen 30, 38, 40 und 42, wobei die Seitenflächen 40 und 42 ein erstes Seitenflächenpaar 44 des Raspelgrundkörpers 12 bilden, die etwas gegeneinander geneigt und in distaler Richtung aufeinander zu verlaufen. Die Seitenflächen 30 und 38 sind, ebenso wie die Seitenflächen 40 und 42 eben und bilden ein zweites Seitenflächenpaar 46. Die Seitenflächen 30 und 38 sind bezüglich einer Längsachse 48 des Schafts 36 und damit auch relativ zueinander ebenfalls geneigt, und zwar um einen Schaftwinkel 50, welcher einen Wert von etwa 7° aufweist. Alternativ sind auch Schaftwinkel 50 im Bereich von 0° bis 15° denkbar. Die Seitenflächen 30 und 38 sind im Wesentlichen bis zu einem distalen Endbereich 52 des Raspelgrundkörpers 12 eben. Insgesamt wird so ein konischer Schaft 36 ausgebildet.

Der distale Endbereich 52 definiert ein distales Ende 54 des Schafts 36 in Form einer stumpfen Spitze 56. Diese ist insbesondere lateralseitig vom Raspelgrundkörper 12 weg weisend konvex gekrümmt.

Die mediale Seitenfläche 38 ist bis etwa auf Höhe des Einschnitts 26 eben und ausgehend von diesem etwas in medialer Richtung gekrümmt, so dass eine vom Raspelgrundkörper 12 weg weisende konkave Krümmung 58 definiert wird. Von einer medialseitigen, etwas in medialer Richtung geneigten Endfläche 60 steht ein ein Kupplungselement bildender Kupplungszapfen 62 ab, welcher in eine in den Figuren nicht im Detail dargestellte Kupplungsaufnahme 64, die ebenfalls ein Kupplungselement einer Kopplungseinrichtung 66 zum lösbaren Koppeln des Raspelgriffs 14 mit dem Raspelgrundkörper 12 definiert, eingreifen kann, um den Raspelgrundkörper 12 mit dem Raspelgriff 14 zu verbinden.

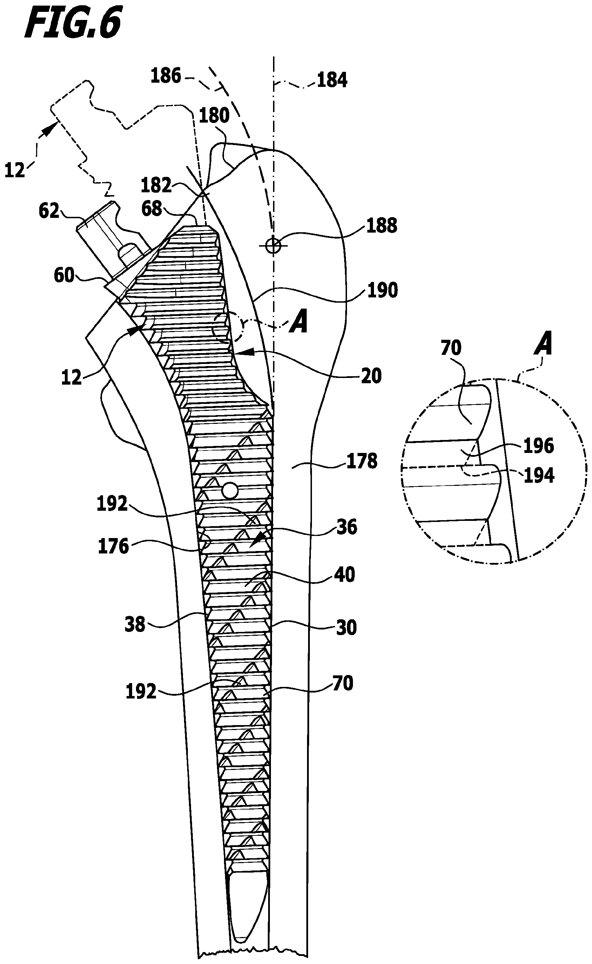

Die Außenkontur 22 definiert eine proximale Endfläche 68 des Raspelkörpers 18, die etwa um 40° gegenüber der Endfläche 60 geneigt ist und quer zur Längsachse 48 verläuft. Sowohl der Raspelgrundkörper 12 als auch der Raspelkörperteil 16 sind auf ihren Außenflächen mit Raspelzähnen 70 versehen, nicht jedoch auf der Raspelkörperteilanlagefläche 32 sowie der Raspelgrundkörperanlagefläche 34. Raspelzahnfrei ist zudem die Spitze 56 sowie die Endflächen 60 und 68. Wie insbesondere in Figur 6 gut zu erkennen, sind die Raspelzähne 70, allerdings nur der Seitenflächen 40 und 42, distalseitig des Einschnitts 26 durch insgesamt sieben parallel zueinander, jedoch relativ zur Längsachse 48 um etwa 45° geneigt verlaufende sogenannte Spanbrechernuten 192 unterbrochen. Sie dienen dazu, den Druck im Markraum 176 beim Präparieren desselben zu reduzieren. Des Weiteren sind die Raspelzähne 70, allerdings nur der Seitenflächen 40 und 42, proximalseitig des Einschnitts 26 atraumatisch ausgebildet. Wie im vergrößerten Ausschnitt A in Figur 6 gut zu erkennen, sind in der Ausschnittsvergrößerung A gestrichelt eingezeichnete Schneidkanten 194 der Raspelzähne 70 der Seitenflächen 40 und 42 proximalseitig des Einschnitts 26 entfernt, so dass die Raspelzähne 70 etwa parallel zu den jeweiligen Seitenflächen 40 beziehungsweise 42 verlaufende Endflächen 196 aufweisen. Die Anzahl der Spanbrechernuten 192 variiert in Abhängigkeit einer Größe des Raspelgrundkörpers 12. Bei kleinen Raspelgrundkörpern 12 sind weniger Spanbrechernuten 192 vorgesehen als bei großen Raspelgrundkörpern 12.

Zum lösbaren Verbinden des Raspelgrundkörpers 12 und des Raspelkörperteils 16 dient eine Verbindungseinrichtung 72, welche ein weibliches Verbindungselement 74 und ein männliches Verbindungselement 76 umfasst, die miteinander formschlüssig in Eingriff bringbar sind. Das weibliche Verbindungselement 74 ist in Form einer Sacklochbohrung 78 ausgebildet, die sich vom proximalen Ende 28 in den Schaft 36 hinein etwas über den Einschnitt 26 hinaus in distaler Richtung erstreckt. Eine Längsachse 80 der Sacklochbohrung 78 ist relativ zur Längsachse 48 um einen Winkel 82 geneigt, der etwa 5° beträgt. Die Sacklochbohrung 78 ist im Bereich ihres proximalen Endes medialseitig durch eine Aussparung 84 erweitert, in welche in der Verbindungsstellung ein korrespondierender Vorsprung 86 des Raspelkörperteils 16 im Wesentlichen formschlüssig eingreift.

Die Sacklochbohrung 78 ist zur Raspelkörperteilanlagefläche 32 hin über einen lateralen Führungsschlitz 88 eröffnet, welcher in der Verbindungsstellung von einem Haltebereich 90 des Raspelkörperteils 16 durchsetzt wird, welcher das männliche Verbindungselement 76 trägt. Dieses ist im Wesentlichen als ein zapfenförmiger Zapfenbereich 94 ausgebildet und erstreckt sich in dieser Form über etwa die halbe Länge der Sacklochbohrung 78 ausgehend von deren distalem Ende 92.

Einstückig mit dem Zapfenbereich 94 des männlichen Verbindungselements 76 ausgebildet ist ein Federabschnitt 96, welcher sich parallel zur Längsachse 80 in proximaler Richtung weisend erstreckt. Er trägt an seinem proximalen Ende einen etwas in medialer Richtung vorstehenden Sicherungsvorsprung 98, welcher einen Teil einer insgesamt mit dem Bezugszeichen 100 bezeichneten Sicherungseinrichtung bildet. Der Sicherungsvorsprung 98 ist somit am Verbindungselement 76 angeordnet beziehungsweise wird von diesem umfasst. Ein weiteres Sicherungselement in Form einer Sicherungsausnehmung 102 ist etwas distalseitig der Aussparung 84 an der Sacklochbohrung 78 ausgebildet und weist in lateraler Richtung. Ist das männliche Verbindungselement mit seinem Zapfenbereich 94 bis an das Ende 92 herangeführt, kann der durch den Federabschnitt 96 federnd vorgespannte Sicherungsvorsprung 98 in die Sicherungsausnehmung 102 eintauchen und auf diese Weise den Raspelkörperteil 16 in der Verbindungsstellung am Raspelgrundkörper 12 automatisch sichern. Die Prothesenraspel nimmt dann die in Figur 4 dargestellte Sicherungsstellung ein. Die Sicherungseinrichtung 100 ist zudem so ausgebildet, dass der Sicherungsvorsprung 98 in die Sicherungsausnehmung 102 eingreift, wenn der Vorsprung 86 in die Aussparung 84 eintaucht.

Der Raspelkörperteil 16 weist ferner ein Kupplungselement 104 in Form einer Kupplungsausnehmung auf, die mit einem korrespondierenden Kupplungsvorsprung 106 eines Handgriffs 108 in Eingriff gebracht werden kann, um den Raspelkörperteil 16 an den Raspelgrundkörper 12 heranzuführen und mit diesem zu verbinden.

Die Prothesenraspel 10 umfasst ferner einen Kastenmeißel 110, welcher einen Meißelteil bildet, der mit dem Raspelgrundkörper 12 verbindbar ist. Der Kastenmeißel 110 umfasst einen im Wesentlichen quaderförmigen Meißelkörper 112, welcher eine Schneidkante 114 im Übergangsbereich zwischen einer distalen Endfläche 116 und einer lateralen Seitenfläche 118 umfasst zur Bearbeitung von Knochen. Somit bildet das proximale Ende 28 des Raspelgrundkörpers 12 einen Anschlag 166 zum Begrenzen einer Bewegung des Kastenmeißels 110 relativ zum Raspelgrundkörper 12 in distaler Richtung.

Von der Endfläche 116 steht ein Kupplungszapfen 120 in proximaler Richtung weisend ab, welcher korrespondierend zur Sacklochbohrung 78 ausgebildet und in diese von proximal her kommend mit seinem distalen Ende 122 voran einschiebbar ist. Am Meißelkörper 112 ist in proximaler Richtung weisend eine Kupplungsausnehmung 124 ausgebildet, welche mit einem korrespondierenden Kupplungsvorsprung 126 eines Handgriffs 128 in Eingriff gebracht werden kann. Damit lässt sich der Kastenmeißel 110 einfach und sicher handhaben und insbesondere mit dem Raspelgrundkörper 12 in einer Bearbeitungsstellung koppeln.

Nachfolgend wird die Raspelkörperteilaufnahme nochmals detaillierter beschrieben.

Die Raspelkörperteilanlagefläche 32 umfasst einen ersten Raspelkörperteilanlageflächenbereich 130, welcher eben beziehungsweise im Wesentlichen eben ist. Dieser erstreckt sich bis hin zum proximalen Ende 28 der Raspelkörperteilaufnahme 20. Der erste Raspelkörperteilanlageflächenbereich 130 und die Längsachse 48 des Raspelgrundkörpers 12 definieren zwischen sich einen Neigungswinkel 132 von etwa 5°. Dieser kann wahlweise auch in einem Bereich von 0° bis 25° liegen.

Die Raspelkörperteilanlagefläche 32 umfasst ferner einen zweiten Raspelkörperteilanlageflächenbereich 134, welcher kontinuierlich gekrümmt ist. Dieser weist bei dem in den Figuren dargestellten Ausführungsbeispiel einen konstanten oder im Wesentlichen konstanten Krümmungsradius 136 auf, der in einem Bereich von 10 mm bis 20 mm liegt. Der zweite Raspelkörperteilanlageflächenbereich 134 erstreckt sich bis zum distalen Ende 24 der Raspelkörperteilaufnahme 20. Die Außenkontur 22 definiert im Bereich des Einschnitts 26 eine erste Tangentialebene 138. Eine zweite Tangentialebene 140 wird definiert durch eine Ebene, die an der vom Raspelgrundkörper 12 weg weisenden Raspelkörperteilanlagefläche 32 im Bereich des distalen Endes 24 der Raspelkörperteilaufnahme 20 anliegt. Die Tangentialebenen 138 und 140 definieren einen Einschnittwinkel 142 zwischen sich, welcher kleiner als 85° ist. Bei dem in den Figuren dargestellten Ausführungsbeispiel beträgt der Einschnittwinkel 142 etwa 60°.

Insgesamt wird so eine stetige oder im Wesentlichen stetige Fläche definiert, nämlich die Raspelkörperteilanlagefläche 32, die lediglich durch den Führungsschlitz 88 unterbrochen ist. Stetig bedeutet hier insbesondere mathematisch stetig, so dass der erste Raspelkörperteilanlageflächenbereich 130 ohne Knick oder Sprung in den zweiten Raspelkörperteilanlageflächenbereich 134 übergeht.

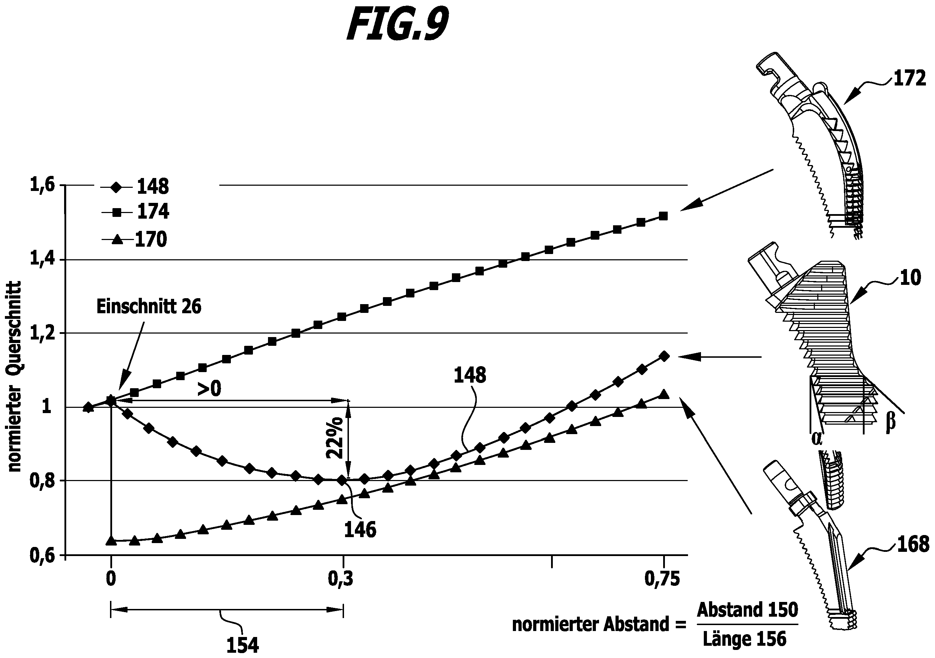

Eine Querschnittsfläche 144 des Raspelgrundkörpers 12 senkrecht zur Längsachse 48 desselben nimmt ausgehend vom distalen Ende 24 der Raspelkörperteilaufnahme 20 in Richtung auf das distale Ende 54 hin ab. Des Weiteren nimmt aber auch eine Querschnittsfläche 144 des Raspelgrundkörpers 12 senkrecht zur Längsachse 48 desselben ausgehend vom distalen Ende 24 der Raspelkörperteilaufnahme 20 in Richtung zum proximalen Ende 28 des Raspelgrundkörpers 12 hin bis zu einem Minimum 146 ab und vom Minimum 146 bis zum proximalen Ende 28 wieder zu. Eine Funktion 148 der normierten Querschnittfläche 144 des Raspelgrundkörpers 12 in Abhängigkeit eines Abstands 150 vom distalen Ende 24 ist in Figur 9 mit 148 bezeichnet. Wie in Figur 9 zu erkennen, bildet die Funktion 148 der Querschnittsfläche 144 des Raspelgrundkörpers 12 senkrecht zur Längsachse 48 ausgehend vom distalen Ende 24 der Raspelkörperteilaufnahme 20 in Richtung auf das proximale Ende 28 des Raspelgrundkörpers hin in Abhängigkeit des Abstandes 150 einer die jeweilige Querschnittsfläche 144 definierenden Schnittebene 152 vom distalen Ende 24 der Raspelkörperteilaufnahme 20 eine mathematisch stetige Funktion. Mathematisch stetig bedeutet, dass die Funktion ableitbar ist und sich keine Sprünge der Ableitungsfunktion ergeben. Das Minimum 146 weist vom distalen Ende 24 der Raspelkörperteilaufnahme 20 einen Abstand 154 auf. Dieser Abstand 154 entspricht mindestens dem 0,1fachen einer Länge 156 der Raspelkörperteilaufnahme 20 parallel zur Längsachse. Bei dem in den Figuren dargestellten Ausführungsbeispiel weist der Abstand 154 einen Wert auf, welcher in etwa mindestens dem 0,3fachen der Länge 156 entspricht.

Die Weiteren schließen die zweite Tangentialebene 140 und der erste Raspelkörperteilanlageflächenbereich 130 einen stumpfen Öffnungswinkel 158 ein. Dieser weist einen Wert in einem Bereich von 95° bis 160° auf, vorzugsweise 105° bis 150°. Bei dem in den Figuren dargestellten Ausführungsbeispiel beträgt der Öffnungswinkel 158 etwa 125°.

Eine Länge 160 des ersten Raspelkörperteilanlageflächenbereichs 130 parallel oder im Wesentlichen parallel zur Längsachse 48 des Raspelgrundkörpers 12 entspricht etwa dem 0,4fachen bis 0,75fachen der Länge 156 der Raspelkörperteilaufnahme 20. Vorzugsweise entspricht die Länge 160 etwa dem 0,6 bis 0,7fachen der Länge 156. Bei dem in den Figuren dargestellten Ausführungsbeispiel entspricht die Länge 160 etwa dem 0,6fachen der Länge 156.

Des Weiteren entspricht die Länge 156 der Raspelkörperteilaufnahme 20 parallel oder im Wesentlichen parallel zur Längsachse 48 des Raspelgrundkörpers 12 etwa dem 0,15 bis 0,4fachen einer Gesamtlänge 162 des Raspelgrundkörpers 12. Vorzugsweise entspricht die Länge 156 etwa dem 0,25 bis etwa 0,3fachen der Gesamtlänge 162. Bei dem in den Figuren dargestellten Ausführungsbeispiel liegt das Verhältnis zwischen der Länge 156 und der Gesamtlänge 162 bei etwa 0,27 bis etwa 0,28.

Im Minimum 146 weist die Querschnittsfläche 144 des Raspelgrundkörpers 12 einen Wert auf, welcher etwa dem 0,5 bis 0,8fachen der Querschnittsfläche 144 im Bereich des Einschnitts 26 entspricht. Bei dem in den Figuren dargestellten Ausführungsbeispiel liegt das Verhältnis bei etwa 0,62 bis 0,7.

Die Raspelkörperteilanlagefläche 32 und die an diese angrenzenden Seitenflächen 40 und 42 weisen abgerundete Kanten 164 auf. Sowohl der Raspelgrundkörper 12 als auch der Raspelkörperteil 16 sind jeweils einstückig ausgebildet.

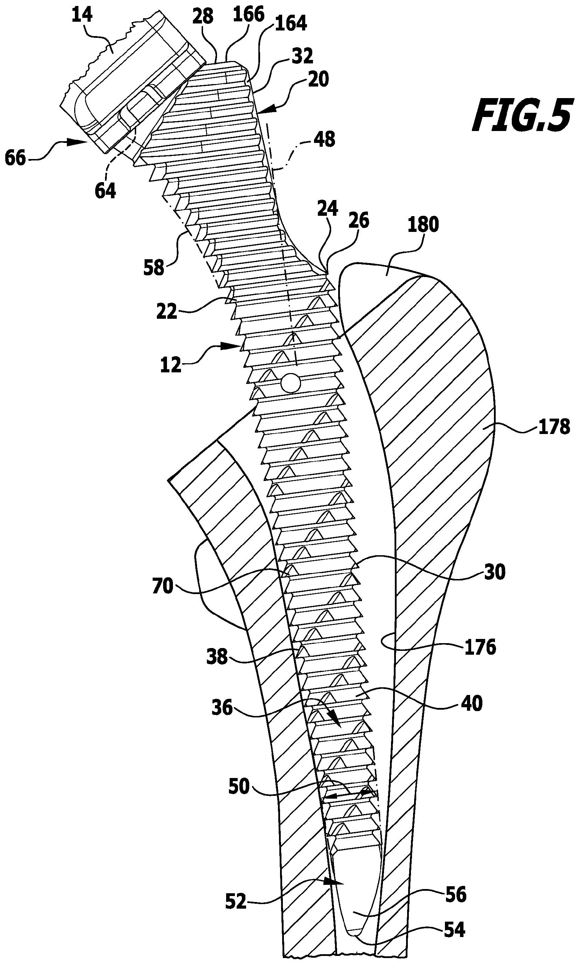

In Figur 9 ist nicht nur die Funktion 148 des normierten Querschnitts in Abhängigkeit des normierten Abstands 150 bezogen auf die Länge 156 dargestellt. Der rechts unten in Figur 9 dargestellten Raspel 168 ist die Funktion 170 zugeordnet. Die Raspel 168 ist in der DE 10 2008 020 192 A1 in Figur 9 dargestellt. Oben rechts in Figur 9 ist die Raspel 172 dargestellt, der die Funktion 174 zugeordnet ist. Die Raspel 172 ist beispielsweise in der DE 10 2008 020 192 A1 in Figur 5 dargestellt.

Die Funktionen 148, 170 und 174 der drei Raspeln 10, 168 und 172 unterscheiden sich signifikant. Bei der Raspel 168 verringert sich der normierte Querschnitt um mehr als 35% direkt im Bereich des Einschnitts 26, was als Sprungfunktion erkennbar ist. Dagegen ist bei der oben beschriebenen Raspel 10 das Minimum 146 sehr weit vom Einschnitt 26 entfernt. Bei der Raspel 172 nimmt die normierte Querschnittsfläche ausgehend vom Einschnitt 26 kontinuierlich zu.

Durch den besonderen Verlauf der Funktion 148 ist es möglich, den Schaft 36 des Raspelkörpers 18 in den Markraum 176 eines Femurs 178 nach teilweiser Resektion des Trochanters 180 einzuführen, ohne dass ein verbleibender, vorspringender Teil 182 des Trochanters 180, welcher in mediale Richtung weist mit dem Raspelgrundkörper 12 in Kontakt treten kann. Die besondere Formgebung der Raspelkörperteilanlagefläche 32 stellt sicher, wie insbesondere in den Figuren 5 und 6 gut zu erkennen, dass unabhängig von einer Einführposition des Raspelgrundkörpers 12 der Teil 182 des Trochanters 180 unberührt bleibt. Entsprechend wird auch umliegendes Weichteilgewebe beim Einführen des Raspelgrundkörpers 12 nicht traumatisiert. Beim Einführen des Schafts 36 in den Markraum 176 gleitet die stumpfe Spitze 56 an der lateralen Kortikalis ab und erzwingt so eine Rotation des Raspelgrundkörpers 12 relativ zu einer vom Femur 178 definierten Achse.

Die Einführung des Raspelgrundkörpers 12 kann zwischen zwei extremen Bahnen erfolgen, die definiert werden durch die jeweils lateralsten Punkte oder Bereiche des Raspelgrundkörpers 12. Bei einer herkömmlichen Raspel liegt dieser Punkt auf der lateralen Seite des Raspelgrundkörpers, und zwar im Schnittpunkt 188 der in Figur 6 gestrichelt eingezeichneten Bahnkurven 184 und 186. Die Bahnkurve 184 verläuft parallel zur Längsachse 48. Je nach lateraler Ausladung einer solchen Raspel werden bei der Bahnkurve 184 der Trochanter 180 und die Weichteile bei den einzelnen Raspelgängen mehr oder weniger in Mitleidenschaft gezogen. Die andere extreme Bahnkurve 186 verläuft soweit medial wie möglich. Die Form der Bahnkurve 186 wird bestimmt durch die Länge der Raspel, deren Spitzenform, der medialen Form der Raspel sowie der Knochenform. Auf dieser Bahnkurve 186 werden sowohl die Weichteile als auch der Trochanterbereich deutlich weniger in Mitleidenschaft gezogen. Daher sollte das Raspeln soweit wie möglich auf der Bahnkurve 186 erfolgen. Allerdings wird sich in einer klinischen Anwendung der tatsächliche Verlauf der Bahnkurve zwischen den beiden beschriebenen Bahnkurven 184 und 186 bewegen.

Beim Einschlagen einer Raspel bewegt sich deren laterale Seite auf einem großen Teil einer Raspelbahn an der Kortikalis des Trochanters 180 entlang. Da dieser Knochen sehr hart ist, wird die Raspel 10, unabhängig davon ob lateral Raspelzähne 70 angebracht sind oder nicht, nach medial gedrückt. Der Anwender muss, um eine varische Fehlpositionierung zu vermeiden, entsprechend dagegendrücken. Dies wiederum führt zu einer eher lateral verlaufenden Raspelbahn. Ist die Schulter der Raspel an der Kortikalis vorbei, ist der verbleibende Weg zu gering, um eine eventuell schon nach medial verkippte Raspel noch aufzurichten, bevor sie ihre endgültige Position erreicht. Letztendlich hat der Anwender beim Einsatz von aus dem Stand der Technik bekannten Raspeln die Wahl zwischen einer atraumatischen medial verlaufenden Bahnkurve 186, bei der er eine varische Fehlpositionierung riskiert, oder einer traumatischen lateralen Bahnkurve 184, bei der er eine Fehlpositionierung vermeidet.

Bei der oben beschriebenen Raspel 10 treten diese Probleme im Wesentlichen nicht auf. Durch die besonders große Raspelkörperteilaufnahme 20 und deren oben beschriebene besondere Formgebung wird erreicht, dass sowohl der Trochanter 180 als auch umliegende Weichteile in diesem Bereich erheblich geschont und eine varische Fehlpositionierung vermieden werden. Durch das Vorsehen der Raspelkörperteilaufnahme 20 wird der lateralste Punkt des Raspelgrundkörpers 12 gebildet durch die Kante zwischen Raspelkörperteilanlagefläche 32 und der Seitenfläche 30 im Bereich des Einschnitts 26. Die so definierte Bahnkurve 190 liegt weit unterhalb der optimalen Bahnkurve 186, die mit herkömmlichen Raspeln erreichbar ist.

Bei der Raspel 10 liegt beim Einführen derselben in den Markraum 176 nur die Spitze 56 an der Kortikalis im Trochanterbereich an, also distalseitig der Raspelkörperteilaufnahme 20. Sobald die Spitze 56 an der Kortikalis vorbei ist, besteht kein Kontakt mehr zwischen dem Raspelgrundkörper 12 und der Kortikalis. Damit lässt auch der Druck auf den Raspelgrundkörper 12 nach. Der verbleibende Weg zum Aufrichten des Raspelgrundkörpers 12 wird durch das Vorsehen der besonderen Raspelteilaufnahme 20 erheblich vergrößert, so dass der Raspelgrundkörper 12 durch den Schaft 36 nahezu von alleine in die optimale Position geführt wird. Anders als bei herkömmlichen Raspeln muss somit der Raspelgrundkörper 12 nicht durch den Anwender in seine richtige Position gedrückt werden, sondern findet seinen Weg praktisch von alleine.

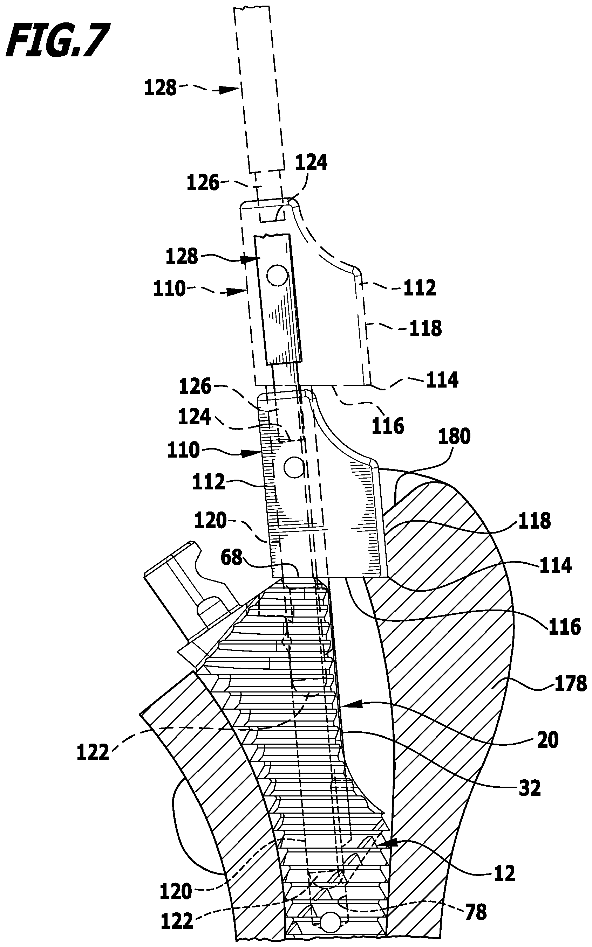

Da nach Einsetzen des Raspelgrundkörpers in den Markraum 176 der Trochanter 180 praktisch nicht bearbeitet ist, wird anschließend mit dem Kasten meißel 110, wie in Figur 7 dargestellt, der Trochanter 180 vorpräpariert und ein Teil desselben entfernt. Der dann noch verbleibende Teil des Trochanters 180 wird mittels des Raspelkörperteils 16 präpariert, und zwar indem dieses mit dem Raspelgrundkörper 12 in der oben beschriebenen Weise verbunden und von proximal her kommend in die Raspelkörperteilaufnahme 20 eingeführt wird. Dabei wird gleichzeitig der noch verbliebene vorspringende Teil 182 des Trochanters 180 in die gewünschte Form gebracht. Der Raspelkörperteil 16 wird dabei ebenso wie der Kastenmeißel 110 durch das weibliche Verbindungselement 74 geführt.

Das weibliche Verbindungselement 74 kann ferner für verschiedene weitere Instrumente als Führung, Ausrichtung oder Orientierung hilfreich sein. Infrage kommen hier insbesondere Sägelehren, Bohrschablonen sowie Instrumente zur Bearbeitung des Knochens in beliebiger Form.

In Abhängigkeit von der Physiologie des Patienten kann die modulare Prothesenraspel 10 auch weitere Raspelkörperteile 16 aufweisen, die in ihrer Form und Größe sich von dem in den Figuren dargestellten Raspelkörperteil 16 unterscheiden können.

## Patentansprüche

1. Modulare Prothesenraspel (10) mit einem Raspelkörper (18), welcher einen Raspelgrundkörper (12) und mindestens einen mit dem Raspelgrundkörper (12) verbindbaren modularen Raspelkörperteil (16) umfasst, wobei der Raspelgrundkörper (12) eine Raspelkörperteilaufnahme (20) aufweist zum Aufnehmen des mindestens einen Raspelkörperteils (16) in einer Verbindungsstellung, in welcher der Raspelgrundkörper (12) und der mindestens eine Raspelkörperteil (16) miteinander verbunden sind und in welcher der Raspelkörper (18) eine teilweise vom Raspelgrundkörper (12) und teilweise vom mindestens einen Raspelkörperteil (16) definierte Außenkontur (22) definiert, wobei eine Verbindungseinrichtung (72) vorgesehen ist zum lösbaren Verbinden des Raspelgrundkörpers (12) und des mindestens einen Raspelkörperteils (16) in der Verbindungsstellung, wobei sich die Raspelkörperteilaufnahme (20) ausgehend von einem ein distales Ende (24) der Raspelkörperteilaufnahme (20) definierenden Einschnitt (26) an einer lateralen Seite (30) des Raspelgrundkörpers (12) bis zu einem proximalen Ende (28) desselben erstreckt, **dadurch gekennzeichnet, dass** die Raspelkörperteilaufnahme (20) seitlich, nämlich in anteriorer und posteriorer Richtung weisend, und proximalseitig geöffnet ist, sodass der Raspelkörperteil (16) die Raspelkörperteilaufnahme (20) in der Verbindungsstellung vollständig ausfüllt, und dass eine erste Tangentialebene (138) der lateralen Seite (30) der Außenkontur (22) im Bereich des Einschnitts (26) und eine zweite Tangentialebene (140) an eine von der Raspelkörperteilaufnahme (20) definierte und vom Raspelgrundkörper (12) weg weisende Raspelkörperteilanlagefläche (32) im Bereich des distalen Endes (24) der Raspelkörperteilaufnahme (20) einen Einschnittwinkel (142) einschließen, welcher kleiner als 85° ist.

2. Prothesenraspel nach Anspruch 1, **dadurch gekennzeichnet, dass** der Einschnittwinkel (142) einen Wert in einem Bereich von 20° bis 70° aufweist, vorzugsweise einen Wert in einem Bereich von 35° bis 55°.

3. Prothesenraspel nach Anspruch 1 oder 2, **dadurch gekennzeichnet, dass** die Raspelkörperteilanlagefläche (32) eine stetige oder im Wesentlichen stetige Fläche definiert.

4. Prothesenraspel nach einem der voranstehenden Ansprüche, **dadurch gekennzeichnet, dass** der mindestens eine Raspelkörperteil (16) eine Raspelgrundkörperanlagefläche (34) aufweist, welche in der Verbindungsstellung flächig oder im Wesentlichen flächig an der Raspelkörperteilanlagefläche (32) anliegt.

5. Prothesenraspel nach einem der voranstehenden Ansprüche, **dadurch gekennzeichnet, dass** die Raspelkörperteilanlagefläche (32) einen ebenen oder im Wesentlichen ebenen ersten Raspelkörperteilanlageflächenbereich (130) umfasst.

6. Prothesenraspel nach Anspruch 5, **dadurch gekennzeichnet, dass** sich der erste Raspelkörperteilanlageflächenbereich (130) hin zu einem proximalen Ende (28) der Raspelkörperteilaufnahme (20) erstreckt.

7. Prothesenraspel nach Anspruch 5 oder 6, **dadurch gekennzeichnet, dass** der erste Raspelkörperteilanlageflächenbereich (130) und eine Längsachse (48) des Raspelgrundkörpers (12) einen Neigungswinkel definieren, welcher einen Wert in einem Bereich von 0° bis 25°, vorzugsweise 0° bis 10°, insbesondere 5°.

8. Prothesenraspel nach einem der voranstehenden Ansprüche, **dadurch gekennzeichnet, dass** die Raspelkörperteilanlagefläche (32) einen zweiten Raspelkörperteilanlageflächenbereich (134) umfasst, welcher kontinuierlich gekrümmt ist.

9. Prothesenraspel nach Anspruch 8, **dadurch gekennzeichnet, dass** der zweite Raspelkörperteilanlageflächenbereich (134) einen konstanten oder im Wesentlichen konstanten Krümmungsradius (136) aufweist.

10. Prothesenraspel nach Anspruch 8 oder 9, **dadurch gekennzeichnet, dass** sich der zweite Raspelkörperteilanlageflächenbereich (134) bis zum distalen Ende (24) der Raspelkörperteilaufnahme (20) erstreckt.

11. Prothesenraspel nach einem der voranstehenden Ansprüche, **dadurch gekennzeichnet, dass** eine Querschnittsfläche (144) des Raspelgrundkörpers (12) senkrecht zu einer Längsachse (48) desselben ausgehend vom distalen Ende (24) der Raspelkörperteilaufnahme (20) in Richtung zu einem proximalen Ende (28) des Raspelgrundkörpers (12) hin bis zu einem Minimum (146) abnimmt und vom Minimum (146) bis zum proximalen Ende (28) hin wieder zunimmt.

12. Prothesenraspel nach Anspruch 11, **dadurch gekennzeichnet, dass** eine Funktion (148) der Querschnittsfläche (144) des Raspelgrundkörpers (12) senkrecht zur Längsachse (48) ausgehend vom distalen Ende (24) der Raspelkörperteilaufnahme (20) in Richtung zu einem proximalen Ende (28) des Raspelgrundkörpers (12) hin in Abhängigkeit des Abstandes (150) der die Querschnittsfläche (144) definierenden Schnittebene (152) vom distalen Ende (24) der Raspelkörperteilaufnahme (20) eine mathematisch stetige Funktion (148) bildet.

13. Prothesenraspel nach einem der voranstehenden Ansprüche, **dadurch gekennzeichnet, dass** eine Verbindungseinrichtung (72) zum lösbaren Verbinden des Raspelgrundkörpers (12) und des mindestens einen Raspelkörperteils (16) in der Verbindungsstellung vorgesehen ist, dass die Verbindungseinrichtung (72) erste und zweite Verbindungselemente (72, 76) umfasst, welche einerseits am Raspelgrundkörper (12) und andererseits am mindestens einen Raspelkörperteil (16) angeordnet oder ausgebildet sind und in der Verbindungsstellung miteinander in Eingriff stehen, dass der Raspelgrundkörper (12) ein weibliches oder im Wesentlichen weibliches Verbindungselement (74) umfasst und dass der mindestens eine Raspelkörperteil (16) ein korrespondierendes männliches oder im Wesentlichen männliches Verbindungselement (76) umfasst.

14. Prothesenraspel nach einem der Ansprüche 5 bis 13, **dadurch gekennzeichnet, dass** die zweite Tangentialebene (140) und der erste Raspelkörperteilanlageflächenbereich (130) einen stumpfen Öffnungswinkel (158) einschließen.

15. Prothesenraspel nach einem der voranstehenden Ansprüche, **gekennzeichnet durch** einen mit dem Raspelgrundkörper (12) lösbar verbindbaren Meißelteil (110).

## Claims

1. Modular prosthesis rasp (10) with a rasp body (18) comprising a rasp main body (12) and at least one modular rasp body part (16) connectable to the rasp main body (12), the rasp main body (12) comprising a rasp body part receptacle (20) for receiving the at least one rasp body part (16) in a connected position in which the rasp main body (12) and the at least one rasp body part (16) are connected to each other, and in which the rasp body (18) defines an outer contour (22) defined partly by the rasp main body (12) and partly by the at least one rasp body part (16), a connecting device (72) being provided for releasably connecting the rasp main body (12) and the at least one rasp body part (16) in the connected position, and the rasp body part receptacle (20) extending from an indentation (26), defining a distal end (24) of the rasp body part receptacle (20), on a lateral side (30) of the rasp main body (12) to a proximal end (28) of the rasp main body, **characterized in that** the rasp body part receptacle (20) is open at the sides, namely facing in the anterior and posterior directions, and proximally, so that the rasp body part (16) fills out the rasp body part receptacle (20) completely in the connected position, and **in that** a first tangential plane (138) of the lateral side (30) of the outer contour (22) in the region of the indentation (26) and a second tangential plane (140) at a rasp body part contact surface (32), defined by the rasp body part receptacle (20) and facing away from the rasp main body (12), in the region of the distal end (24) of the rasp body part receptacle (20) include an indentation angle (142) which is less than 85°.

2. Prosthesis rasp in accordance with claim 1, **characterized in that** the indentation angle (142) has a value ranging from 20° to 70°, preferably a value ranging from 35° to 55°.

3. Prosthesis rasp in accordance with claim 1 or 2, **characterized in that** the rasp body part contact surface (32) defines a continuous or substantially continuous surface.

4. Prosthesis rasp in accordance with any one of the preceding claims, **characterized in that** the at least one rasp body part (16) has a rasp main body contact surface (34), which in the connected position lies with surface-to-surface contact or substantially with surface-to-surface contact against the rasp body part contact surface (32).

5. Prosthesis rasp in accordance with any one of the preceding claims, **characterized in that** the rasp body part contact surface (32) has a plane or substantially plane first rasp body part contact surface region (130).

6. Prosthesis rasp in accordance with claim 5, **characterized in that** the first rasp body part contact surface region (130) extends towards a proximal end (28) of the rasp body part receptacle (20).

7. Prosthesis rasp in accordance with claim 5 or 6, **characterized in that** the first rasp body part contact surface region (130) and a longitudinal axis (48) of the rasp main body (12) define an angle of inclination which has a value ranging from 0° to 25°, preferably 0° to 10°, in particular, 5°.

8. Prosthesis rasp in accordance with any one of the preceding claims, **characterized in that** the rasp body part contact surface (32) has a second rasp body part contact surface region (134) which is continuously curved.

9. Prosthesis rasp in accordance with claim 8, **characterized in that** the second rasp body part contact surface region (134) has a constant or substantially constant radius of curvature (136).

10. Prosthesis rasp in accordance with claim 8 or 9, **characterized in that** the second rasp body part contact surface region (134) extends as far as the distal end (24) of the rasp body part receptacle (20).

11. Prosthesis rasp in accordance with any one of the preceding claims, **characterized in that** a cross-sectional area (144) of the rasp main body (12) perpendicular to a longitudinal axis (48) of the rasp main body, starting from the distal end (24) of the rasp body part receptacle (20), decreases in the direction towards a proximal end (28) of the rasp main body (12) up to a minimum (146) and increases again from the minimum (146) up to the proximal end (28).

12. Prosthesis rasp in accordance with claim 11, **characterized in that** a function (148) of the cross-sectional area (144) of the rasp main body (12) perpendicular to the longitudinal axis (48), starting from the distal end (24) of the rasp body part receptacle (20) in the direction towards a proximal end (28) of the rasp main body (12), forms, in dependence upon the distance (150) of the cutting plane (152) defining the cross-sectional area (144) from the distal end (24) of the rasp body part receptacle (20), a mathematically continuous function (148).

13. Prosthesis rasp in accordance with any one of the preceding claims, **characterized in that** a connecting device (72) is provided for releasably connecting the rasp main body (12) and the at least one rasp body part (16) in the connected position, **in that** the connecting device (72) comprises first and second connecting elements (72, 76), which are arranged or formed, on the one hand, on the rasp main body (12) and, on the other hand, on the at least one rasp body part (16) and are in engagement with each other in the connected position, **in that** the rasp main body (12) comprises a female or substantially female connecting element (74), and **in that** the at least one rasp body part (16) comprises a corresponding male or substantially male connecting element (76).

14. Prosthesis rasp in accordance with any one of claims 5 to 13, **characterized in that** the second tangential plane (140) and the first rasp body part contact surface region (130) form an obtuse included angle (158).

15. Prosthesis rasp in accordance with any one of the preceding claims, **characterized by** a chisel part (110), which is releasably connectable to the rasp main body (12).

## Revendications

1. Râpe de prothèse (10) modulaire comportant, un corps de râpe (18), qui comprend un corps de base de râpe (12) et au moins une partie de corps de râpe modulaire (16) pouvant être reliée au corps de base de râpe (12), râpe de prothèse
dans laquelle le corps de base de râpe (12) présente un logement d'accueil de partie de corps de râpe (20) destiné à accueillir ladite au moins une partie de corps de râpe (16) dans une position de liaison dans laquelle le corps de base de râpe (12) et ladite au moins une partie de corps de râpe (16) sont reliés mutuellement et dans laquelle le corps de râpe (18) définit un contour extérieur (22) défini partiellement par le corps de base de râpe (12) et partiellement par ladite au moins une partie de corps de râpe (16),
dans laquelle il est prévu un dispositif de liaison (72) pour relier de manière amovible le corps de base de râpe (12) et ladite au moins une partie de corps de râpe (16) dans la position de liaison, et
dans laquelle le logement d'accueil de partie de corps de râpe (20) s'étend à partir d'une incision (26) sur un côté latéral (30) du corps de base de râpe (12), qui définit une extrémité distale (24) du logement d'accueil de partie de corps de râpe (20), jusqu'à une extrémité proximale (28) de celui-ci,
**caractérisée en ce que** le logement d'accueil de partie de corps de râpe (20) est latéral, à savoir dirigé dans la direction antérieure et postérieure, et est ouvert du côté proximal, de sorte que la partie de corps de râpe (16) remplit complètement le logement d'accueil de partie de corps de râpe (20) dans la position de liaison, et **en ce qu'**un premier plan tangentiel (138) au côté latéral (30) du contour extérieur (22) dans la zone de l'incision (26), et un deuxième plan tangentiel (140) à une surface d'appui de partie de corps de râpe (32), qui est définie par le logement d'accueil de partie de corps de râpe (20) et orientée dans une direction s'écartant du corps de base de râpe (12), dans la zone de l'extrémité distale (24) du logement d'accueil de partie de corps de râpe (20), forment un angle d'incision (142) qui est inférieur à 85°.

2. Râpe de prothèse selon la revendication 1, **caractérisée en ce que** l'angle d'incision (142) présente une valeur se situant dans une plage de 20° à 70°, de préférence une valeur se situant dans une plage de 35° à 55°.

3. Râpe de prothèse selon la revendication 1 ou la revendication 2, **caractérisée en ce que** la surface d'appui de partie de corps de râpe (32) définit une surface continue ou sensiblement continue.

4. Râpe de prothèse selon l'une des revendications précédentes, **caractérisée en ce que** ladite au moins une partie de corps de râpe (16) présente une surface d'appui de corps de base de râpe (34), qui, dans la position de liaison, s'appuie par un appui surfacique ou sensiblement surfacique sur la surface d'appui de partie de corps de râpe (32).

5. Râpe de prothèse selon l'une des revendications précédentes, **caractérisée en ce que** la surface d'appui de partie de corps de râpe (32) comprend une première zone de surface d'appui de partie de corps de râpe (130) plane ou sensiblement plane.

6. Râpe de prothèse selon la revendication 5, **caractérisée en ce que** la première zone de surface d'appui de partie de corps de râpe (130) s'étend en direction d'une extrémité proximale (28) du logement d'accueil de partie de corps de râpe (20).

7. Râpe de prothèse selon la revendication 5 ou la revendication 6, **caractérisée en ce que** la première zone de surface d'appui de partie de corps de râpe (130) et un axe longitudinal (48) du corps de base de râpe (12) définissent un angle d'inclinaison présentant une valeur se situant dans une plage de 0° à 25°, de préférence de 0° à 10°, et présentant notamment une valeur de 5°.

8. Râpe de prothèse selon l'une des revendications précédentes, **caractérisée en ce que** la surface d'appui de partie de corps de râpe (32) comprend une deuxième zone de surface d'appui de partie de corps de râpe (134), qui est d'une courbure continue.

9. Râpe de prothèse selon la revendication 8, **caractérisée en ce que** la deuxième zone de surface d'appui de partie de corps de râpe (134) présente un rayon de courbure (136) constant ou sensiblement constant.

10. Râpe de prothèse selon la revendication 8 ou la revendication 9, **caractérisée en ce que** la deuxième zone de surface d'appui de partie de corps de râpe (134) s'étend jusqu'à l'extrémité distale (24) du logement d'accueil de partie de corps de râpe (20).

11. Râpe de prothèse selon l'une des revendications précédentes, **caractérisée en ce qu'**une surface de section transversale (144) du corps de base de râpe (12) perpendiculairement à un axe longitudinal (48) de celui-ci, diminue à partir de l'extrémité distale (24) du logement d'accueil de partie de corps de râpe (20) en direction d'une extrémité proximale (28) du corps de base de râpe (12), jusqu'à un minimum (146), puis augmente à nouveau à partir du minimum (146) jusqu'à l'extrémité proximale (28).

12. Râpe de prothèse selon la revendication 11, **caractérisée en ce qu'**une fonction (148) de la surface de section transversale (144) du corps de base de râpe (12) perpendiculairement à l'axe longitudinal (48), par rapport à la distance (150) entre le plan de coupe (152) définissant la surface de section transversale (144) et l'extrémité distale (24) du logement d'accueil de partie de corps de râpe (20), est, à partir de l'extrémité distale (24) du logement d'accueil de partie de corps de râpe (20) en direction d'une extrémité proximale (28) du corps de base de râpe (12), une fonction mathématique continue (148).

13. Râpe de prothèse selon l'une des revendications précédentes, **caractérisée en ce qu'**il est prévu un dispositif de liaison (72) pour assurer la liaison amovible du corps de base de râpe (12) et de ladite au moins une partie de corps de râpe (16) dans la position de liaison, **en ce que** le dispositif de liaison (72) comprend des premier et deuxième éléments de liaison (72, 76), qui sont agencés et/ou formés d'une part sur le corps de base de râpe (12) et d'autre part sur ladite au moins une partie de corps de râpe (16), et qui sont en prise réciproque dans la position de liaison, **en ce que** le corps de base de râpe (12) comporte un élément de liaison (74) femelle ou sensiblement femelle, et **en ce que** ladite au moins une partie de corps de râpe (16) comporte un élément de liaison (76) mâle ou sensiblement mâle, correspondant.

14. Râpe de prothèse selon l'une des revendications 5 à 13, **caractérisée en ce que** le deuxième plan tangentiel (140) et la première zone de surface d'appui de partie de corps de râpe (130) forment un angle d'ouverture (158) obtus.

15. Râpe de prothèse selon l'une des revendications précédentes, **caractérisée par** une pièce de ciseau ou burin (110) pouvant être reliée de manière amovible au corps de base de râpe (12).
